# EUROPEAN PATENT APPLICATION

(11) **EP 2 047 859 A1**
(43) Date of publication of application: **15.04.2009**
(21) Application number: 07744855.3
(22) Date of filing: 07.06.2007
(51) Int. Cl.: A61K 38/22, A61K 35/14, A61P 9/00, A61P 9/10, A61P 43/00

(54) **TREATMENT OF ISCHEMIC DISEASE USING ERYTHROPOIETIN**

(30) Priority: 07.06.2006 JP 2006158998
(71) Applicant: The University of Tokushima, Tokushima-shi, Tokushima 770-8501 (JP); CHUGAI SEIYAKU KABUSHIKI KAISHA, Tokyo, 115-8543 (JP)
(72) Inventor: MATSUMOTO, Toshio, Tokushima-shi, Tokushima 770-8501 (JP); KITAGAWA, Tetsuya, Tokushima-shi, Tokushima 770-8501 (JP); ABE, Masahiro, Tokushima-shi, Tokushima 770-8501 (JP); AKAIKE, Masashi, Tokushima-shi, Tokushima 770-8503 (JP); NAGAYOSHI, Kazunari, Gotenba-shi, Shizuoka 412-8513 (JP); YAMAMOTO, Kaname, Gotenba-shi, Shizuoka 412-8513 (JP); HIGUCHI, Masato, Gotenba-shi, Shizuoka 412-8513 (JP)
(74) Representative: Vossius & Partner
(86) International application number: PCT/JP2007/061525
(87) International publication number: WO 2007/142288

(57) **Abstract**

Disclosed is a method for stimulating revascularization in a subject comprising the steps of:
(a) administering erythropoietin to the subject;
(b) collecting peripheral blood mononuclear cells from the subject; and
(c) administering the collected peripheral blood mononuclear cells to a target site of the subject. Peripheral blood mononuclear cells, and particularly CD34-positive cells, are mobilized into the peripheral blood of a subject by the administration of erythropoietin to the subject. The method of the present invention is useful for the treatment of ischemic diseases, such as peripheral vascular disorder.

## Description

### Field of the Invention

The present invention relates to a method of revascularization, a method for treating ischemic diseases, and a composition used for these methods.

### Background of the Invention

Peripheral vascular disorder is a medical condition in which peripheral tissue becomes an ischemic state due to a reduced peripheral arterial blood flow caused by, for example, constriction of the vessel lumen, development of blood clots, vessel occlusion, vasculitis, vessel shrinkage, or an increase in blood viscosity. In recent years it has been discovered that blood vessel neogenesis is enhanced when bone marrow-derived mononuclear cells are transplanted to an ischemic site, and bone marrow-derived mononuclear cell transplantation is believed to be a potential method for treating peripheral vascular disorder. However, the transplantation of bone marrow-derived mononuclear cells requires collecting bone marrow from the patient, which imposes a substantial burden on the patient.

It has been attempted to use peripheral blood-derived mononuclear cells in place of bone marrow-derived mononuclear cells in order to lessen the burden on the patient; however, this method has not been successful in providing an effective therapy due to the small number of mononuclear cells present in the peripheral blood.

Erythropoietin (also referred to as EPO) is an acidic glycoprotein hormone that promotes the differentiation and proliferation of erythroid progenitor cells and is produced mainly in the kidney. Erythrocytes, the most abundant cells in the blood, will function for a certain period of time and then be destroyed mainly in the spleen (the average life span in humans is about 120 days). Under normal conditions the total peripheral erythrocyte count is continuously held constant by continuous supply from the bone marrow. EPO plays a central role in this homeostasis of erythrocytes in the body. In clinical settings, EPO is used to treat anemia and for pre- and post-surgical management.

In addition, it has been reported that EPO has an angiogenesis-promoting activity and is effective as a therapeutic agent for ischemic diseases (Besarab A et al., The New England Journal of Medicine, 339(9), 584-590, (1998), Heeschen C et al., Blood,102(4), 1340-1346, (2003), Bahlmann F H et al., Blood,103(3), 921-926, (2004), Smith K J et al., Cardiovascular Research, (59), 538-548, (2003), Bahlmann F H et al., Kidney International, 64, 1648-1652, (2003)). It has also been reported that EPO promotes the mobilization of vascular endothelial progenitor cells into the peripheral blood (Heeschen C et al., Blood, 102(4), 1340-1346, (2003), Bahlmann F H et al., Blood, 103(3), 921-926, (2004)).

It has been unclear, however, as to whether the cells mobilized into the peripheral blood by EPO are effective for the treatment of ischemic diseases such as peripheral vascular disorders.

### SUMMARY OF THE INVENTION

The present inventors have discovered that the mobilization of mononuclear cells into the peripheral blood is promoted by the administration of EPO and that the mononuclear cells thus mobilized by EPO are particularly useful for the treatment of ischemic diseases.

The present invention provides a composition for mobilizing mononuclear cells for use in the treatment of ischemic diseases or in stimulating revascularization into peripheral blood, wherein the composition comprises erythropoietin as an active ingredient. Preferably the mobilized mononuclear cells are collected from a subject and then administered to the same subject.

Preferably, the composition of the present invention is administered to a subject from 3 to 12 days prior to collecting peripheral blood from the subject.

The present invention also provides a composition for the treatment of ischemic diseases and a composition for stimulating revascularization, comprising as an active ingredient mononuclear cells separated from peripheral blood from a subject to whom erythropoietin has previously been administered. In such compositions, the erythropoietin is preferably administered from 3 to 12 days prior to collecting peripheral blood.

The present invention also provides a composition for mobilizing CD34-positive cells for use in the treatment of ischemic diseases or in stimulating revascularization into peripheral blood comprising erythropoietin as an active ingredient.

In another aspect, the present invention provides a method for preparing mononuclear cells for stimulating revascularization and for the treatment of ischemic diseases, comprising separating mononuclear cells from peripheral blood from a subject to whom erythropoietin has previously been administered.

In another aspect, the present invention provides a method for treating an ischemic disease or stimulating revascularization in a subject comprising the steps of:
(a) administering erythropoietin to the subject;
(b) collecting peripheral blood mononuclear cells from the subject; and
(c) administering the collected peripheral blood mononuclear cells to a target site of the subject.

### BRIEF DESCRIPTION OF THE DRAWINGS

Fig. 1 shows the results of FACS analysis that measured the mobilization of hematopoietic stem cells by erythropoietin;
Fig. 2 shows the results of measurement of the number of colonies of hematopoietic stem cells mobilized by the administration of erythropoietin; and
Fig. 3 shows an angiogram, at one month after the transplantation of peripheral blood mononuclear cells, for a patient (case 3) that had received erythropoietin.

### DESCRIPTION OF THE PREFERRED EMBODIMENTS

### Mobilization of peripheral blood mononuclear cells by erythropoietin

One aspect of the present invention relates to a composition for mobilization of the mononuclear cells for use in the treatment of ischemic diseases or in stimulating revascularization into the peripheral blood, wherein the composition comprises erythropoietin as an active ingredient. The terms "mobilizing" and "mobilization of" the mononuclear cells denote stimulating differentiation and proliferation of multipotent stem cells present in various organs and releasing mononuclear cells comprising multipotent stem cells into blood. The mononuclear cells mobilized in this manner may be collected from the peripheral blood of the subject and administered to the subject in order to treat ischemic diseases or to stimulate revascularization. The subject is preferably a patient suffered from an ischemic disease or a patient in need of a revascularization therapy.

There are no particular limitations in the present invention on the number of doses of erythropoietin administrated to the subject prior to collecting the peripheral blood mononuclear cells, but generally from one to three doses. In the case that three doses are administrated, for example, the first erythropoietin administration is given systemically (for example, subcutaneous or intravenous injection) to the subject about two weeks prior to collection of the peripheral blood mononuclear cells. About one week later the second erythropoietin administration is given systemically after blood donation (blood draw). Another week later, the third erythropoietin administration is given locally, and then the desired peripheral mononuclear cells may be collected from the peripheral blood of the subject. In the case that two doses are administrated, for example, the first erythropoietin administration is given to the subject systemically about one week prior to collection of the peripheral blood mononuclear cells and the second erythropoietin administration is given locally about one week later, and then the desired peripheral mononuclear cells may be collected from the peripheral blood. In the case of a single administration, for example, erythropoietin is administered systemically to the subject and the desired peripheral mononuclear cells can be collected about one week later.

A unit dose of erythropoietin generally contains 1000 U/body to 100000 U/body and preferably is 3000 U/body to 12000 U/body (for example, 6000 U/body). The dose for the individual subject will be determined by the attending physician considering, for example, the age, weight and condition of the subject, and the route of administration. Accordingly, the erythropoietin dose is not limited in the present invention to the doses noted above.

Erythropoietin is generally administered by a parenteral route, for example, it can be administered as an injectable (e.g., subcutaneous, intravenous, intramuscular, intraperitoneal) or by a percutaneous, transmucosal, nasal or pulmonary route. It can also be administered orally.

Peripheral blood mononuclear cells are mononuclear cells present in the peripheral blood. Mononuclear cells, also known as monocytes, are cells that are present mainly in the blood and that reside in a stage of differentiation for macrophage-type cells. Hematopoietic stem cells differentiate in the bone marrow to monoblasts and promonocytes and then to mononuclear cells, which are released into the blood. Upon migration into the tissues, the mononuclear cells differentiate into, for example, macrophages, dendritic cells, and histiocytes.

With regard to the mobilization of mononuclear cells into the peripheral blood using EPO, it has been thought that two weeks are required after EPO administration for mobilization of the mononuclear cells into the peripheral blood (Bahlmann F H et al., Blood, 103(3), 921-926, (2004)). However, it has been discovered in accordance with the present invention that mononuclear cells are mobilized in sufficient quantities for use in the treatment of ischemic diseases or in stimulating revascularization into the peripheral blood at about one week after the administration of EPO.

Accordingly, the present invention relates to a method of obtaining mononuclear cells or a method of preparing mononuclear cells for transplantation, comprising the steps of:
(a) administering erythropoietin to a subject; and
(b) collecting peripheral blood mononuclear cells from the subject about one week after the erythropoietin administration.

In the context of the present invention, the phrase "about one week" is generally from 3 to 12 days, and preferably from 5 to 9 days (for example, from 6 to 8 days, or 7 days).

In another aspect, the present invention relates to a composition for mobilizing CD34-positive cells for use in the treatment of ischemic diseases or in stimulating revascularization into the peripheral blood comprising erythropoietin as an active ingredient.

CD34 is a blood stem cell antigen. It is expressed on blood stem cells, and is also expressed in, for example, vascular endothelial cells, vascular endothelial progenitor cells, and stromal cells. Since CD34-positive cells, such as vascular endothelial progenitor cells, are known to be involved in angiogenesis, the proportion of CD34-positive cells in a transplanted mononuclear cell population is desirably increased in order to achieve a better therapeutic result of ischemic diseases or a better revascularization effect. A population of the mononuclear cells mobilized into the peripheral blood according to the method of the present invention generally has a CD34-positive cell titer sufficient for achieving a therapeutic result of ischemic diseases or a revascularization effect. It is particularly useful in the transplantation with the aim of treating ischemic diseases or stimulating revascularization to increase the proportion of CD34-positive cells by isolating or concentrating the CD34-positive cells from the population of the mononuclear cells obtained by the method of the present invention.

In the present invention, a high proportion of CD34-positive cells in the mononuclear cells means that CD34-positive cells account for at least 1%, preferably at least 2%, and more preferably at least 3% of the mononuclear cells. The upper limit on the proportion of CD34-positive cells in the mononuclear cells can be, for example, 99.99%, 99.9%, or 99% and is theoretically 100%.

The CD34-positive cells present in the mononuclear cells obtained according to the present invention may also be CD45-positive. CD45 is a leukocyte common antigen and is a key membrane glycoprotein of hematopoietic-type cells.

The peripheral blood mononuclear cells can be collected from the subject by a common method. For example, the mononuclear cells can be obtained by directly recovering the blood-derived mononuclear cells by blood apheresis, removing a substantial portion of the erythrocytes, granulocytes, and platelets by centrifugation as necessary to capture the peripheral blood leukocytes, and then washing the obtained peripheral blood leukocytes by, for example, centrifugation.

The peripheral blood mononuclear cells obtained in this manner may optionally be further processed by addition, isolation, or purification. For example, desired cells, such as CD34-positive cells and/or vascular endothelial progenitor cells, may further be concentrated or isolated from the collected peripheral blood mononuclear cells for administration. Substantially pure CD34-positive cells can be prepared using standard techniques. For example, the peripheral blood mononuclear cells are reacted with anti-CD34 antibody, and then the CD34-positive cells are attached to magnetic beads carrying anti-mouse IgG antibody. The CD34-positive cells bound to the magnetic beads are collected with a sheet magnet, and subsequently the CD34-positive cells may be released from the magnetic beads by an enzyme treatment. Alternatively, the CD34-positive cells are bound to an anti-CD34 antibody labeled with a fluorescent dye and collected using a fluorescent cell sorter.

### Treatment of ischemic diseases and revascularization

The peripheral blood mononuclear cells prepared in accordance with the present invention can be administered to the subject for the purpose of treating an ischemic disease. Thus, another aspect the present invention relates to a method for treating an ischemic disease in a subject comprising the steps of:
(a) administering erythropoietin to the subject;
(b) collecting peripheral blood mononuclear cells from the subject; and
(c) administering the collected peripheral blood mononuclear cells to a target site of the subject.

Ischemic disease is a medical condition in which tissue falls into an ischemic state due to a reduced blood flow in the vasculature caused by various factors, such as constriction of the vessel lumen, development of blood clots, vessel occlusion, vasculitis, vessel shrinkage, or an increase in blood viscosity. Ischemic diseases include peripheral vascular disorder, ischemic heart disease (e.g., ischemic cardiomyopathy, myocardial infarction, ischemic heart failure), ischemic cerebrovascular disease, ischemic kidney disease, ischemic lung disease, and ischemic diseases associated with infectious diseases.

Peripheral vascular disorder is a medical condition in which peripheral tissue falls into an ischemic state due to a reduced peripheral arterial blood flow caused by, for example, constriction of the vessel lumen, development of blood clots, vessel occlusion, vasculitis, vessel shrinkage, or an increase in blood viscosity. Diseases associated with peripheral vascular disorder include chronic arterial occlusive diseases such as arteriosclerosis obliterans and Buerger's disease, and progressive systemic sclerosis, systemic erythematosus, Raynaud's disease, vibration syndrome, aneurysm, and vasculitis. Peripheral vascular disorder is a preferred target disease of the therapeutic agent of the present invention, with arteriosclerosis obliterans and Buerger's disease being particularly preferred targets.

In addition, the peripheral blood mononuclear cells prepared in accordance with the present invention can be administered to the subject for the purpose of stimulating revascularization. Thus, another aspect of the present invention relates to a method for stimulating revascularization in a subject comprising the steps of:
(a) administering erythropoietin to the subject;
(b) collecting peripheral blood mononuclear cells from the subject; and
(c) administering the collected peripheral blood mononuclear cells to a target site of the subject.

As used herein, revascularization denotes stimulating angiogenesis and/or the growth and development of blood vessels. The method of the present invention is useful for stimulating neogenesis, growth and development of any type of blood vessels, preferably arteries, and particularly preferably peripheral arteries. Revascularization can be monitored by techniques known to those skilled in the art, for example, by measuring the capillary density using an alkaline phosphatase dye.

As used herein, a target site in general refers to a site where ischemia is occurring and to a site where revascularization is desired. In the case that the transplantation of peripheral blood mononuclear cells to another site will lead revascularization at a site where revascularization is desired, the target site may refer to such another site. Specific examples of the site for local administration include lower limb skeletal muscle, upper limb skeletal muscle, and the heart (heart muscle).

The number of collected mononuclear cells administered or transplanted to the target site is not particularly limited, but is generally from 1.0 × 10⁷ to 1.0 × 10¹² cells, and preferably from 1 × 10⁹ to 1 × 10¹¹ cells.

Local administration is a method that enables efficient administration of the cells to the location of an affected area without exercising significant systemic influence. Local administration can be carried out using, for example, an ordinary syringe, needle, or localized pin.

In the administration of the collected mononuclear cells to the target site, the mononuclear cells may be administered alone or in combination with another substance. The substance co-administered with the mononuclear cells is not particularly limited, but is preferably a substance that enhances the revascularization activity.

### Erythropoietin

Any type of EPO can be employed in the present invention, but is preferably a high-purity EPO, and also preferably those having substantially the same biological activity as mammalian EPO, particularly human EPO.

The EPO used in the present invention may be prepared by any method; for example, it may be natural human EPO obtained by purification from an extract of human origin (see, for example, Japanese Examined Patent Application Publication No. Hei 1-38800) or it may be human EPO produced by genetic engineering techniques in *E. coli,* yeast, Chinese hamster ovary cells (CHO cells), C127 cells, COS cells, myeloma cells, BHK cells, or insect cells, and then extracted, separated, and purified by any of various methods. The EPO used in the present invention is preferably EPO produced by genetic engineering techniques and is preferably EPO produced using mammalian cells (particularly CHO cells) (see, for example, Japanese Examined Patent Application Publication No. Hei 1-44317, Kenneth Jacobs et al., Nature, 313, 806-810 (1985)).

The EPO obtained by genetic recombination techniques may have the same amino acid sequence as EPO of natural origin, or may have the same biological activity as EPO of natural origin but has an amino acid sequence with deletion, substitution, or addition of one or more amino acids. The amino acid deletion, substitution, or addition can be introduced by methods known in the art. For example, those skilled in the art can prepare a polypeptide functionally equivalent to EPO by introducing appropriate mutations in the amino acid sequence of EPO using site-specific mutagenesis (Gotoh, T. et al. (1995) Gene 152, 271-275; Zoller, M. J. and Smith, M. (1983) Methods Enzymol. 100, 468-500; Kramer, W. et al. (1984) Nucleic Acids Res. 12, 9441-9456; Kramer, W. and Fritz, H. J. (1987) Methods Enzymol. 154, 350-367; Kunkel, T. A. (1985) Proc. Natl. Acad. Sci. USA. 82, 488-492; Kunkel (1988) Methods Enzymol. 85, 2763-2766). Amino acid mutations may also be created spontaneously. In general, an amino acid residue is preferably substituted with another amino acid residue that has similar properties of the amino acid side chain. Properties of the amino acid side chain include, for example, hydrophobic amino acids (A, I, L, M, F, P, W, Y, V), hydrophilic amino acids (R, D, N, C, E, Q, G, H, K, S, T), amino acids that have an aliphatic side chain (G, A, V, L, I, P), amino acids that have an hydroxyl-functional side chain (S, T, Y), amino acids that have a sulfur-containing side chain (C, M), amino acids that have a carboxylic acid- or amide-containing side chain (D, N, E, Q), amino acids that have a base-containing side chain (R, K, H), and amino acids that have an aromatic side chain (H, F, Y, W) (the one-letter designations of the amino acids are give in parentheses). It is already known that a polypeptide with a modified amino acid sequence caused by deletion, addition or substitution of one or more amino acid residues retains its biological activity (Mark, D.F. et al., Proc. Natl. Acad. Sci. USA (1984) 81, 5662-5666; Zoller, M.J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413).

A fusion protein of EPO and another protein can also be used. A fusion protein can be constructed, for example, by ligating an EPO-encoding DNA with a DNA coding for another protein in frame; inserting the DNA into an expression vector; which in turn is expressed in a host. Another protein to be fused with the EPO of the present invention is not particularly limited.

Chemically modified EPO can also be used in the invention. Chemically modified EPO include EPO attached to an inorganic or organic compound, e.g., polyethylene glycol or vitamin B12. The EPO used in the present invention may also be an EPO derivative.

As used herein, an EPO derivative refers to EPO with modification of the amino acids in the EPO molecule or EPO with modification of the sugar chains in the EPO molecule. Modification of the sugar chains in the EPO molecule includes the addition, substitution, or deletion of a sugar chain. A preferred sugar chain modification in the present invention is deletion of sialic acid from the EPO molecule.

The EPO produced by recombinant animal cells or urine-derived EPO is generally obtained as an EPO composition that contains various types of EPOs having different sugar chain structures. The number of sialic acids attached to the EPO molecule in an EPO composition will vary among EPO molecules, but in general from 11 to 15 sialic acids are attached to each individual EPO molecule. Asialylated EPO (asialo-EPO) may be prepared by removing the sialic acid. The number of sialic acids to be removed during asialylation process is not particularly limited; all of the sialic acids may be removed, or 1, 2, 3, 4, 5, 6, 7, 8, 9, 10, 11, 12, 13, or 14 sialic acids may be removed. Asialo-EPO preferred for use in the present invention has no more than 10 sialic acids attached to the EPO molecule, more preferably no more than 5, and particularly preferably no more than 2. The number of sialic acids mentioned herein refers to the average number of the sialic acids on the EPO molecules contained in the EPO composition. The average sialic acids per molecule can be measured by methods known to those skilled in the art (see, for example, EP 0428267).

EPO from which sialic acid has been removed (asialo-EPO) can be produced by methods known to those skilled in the art. For example, it can be prepared by treating EPO with an enzyme such as sialidase. A commercially available sialidase may be used for this purpose (see, e.g., National Publication of Translated Version No. 2005-507426; Nobuo Imai et al., Eur. J. Biochem. 194, 457-462 (1990)).

Examples of modification of the amino acids in an EPO molecule include carbamylation, biotinylation, amidination, acetylation, and guanidination. Carbamylation is a preferred amino acid modification in the present invention.

The amino acid residue to be modified is not particularly limited, and may include lysine, arginine, glutamic acid, and tryptophan. Lysine is a preferred amino acid to be modified in the present invention.

Accordingly, EPO containing carbamylated lysine is a particularly preferred embodiment of amino acid-modified EPO in the present invention (see, for example, Marcel L. et al., Derivatives of erythropoietin that are tissue protective but not erythropoietic. Science, 2004; 305:239; Fiordaliso E. et al., A nonerythropoietic derivative of erythropoietin protects the myocardium from ischemia-reperfusion injury. PNAS, 2005; 102:2046). Methods for carbamylation of EPO include, for example, carbamylation by reaction with the cyanate ion, alkylcarbamylation by reaction with alkyl isocyanate, and aryl carbamylation by reaction with aryl isocyanate.

### Pharmaceutical formulations

The EPO can be formulated according to techniques known in the art by appropriate addition of, for example, a suspending agent, solubilizer, stabilizer, isotonizer, preservative, adsorption inhibitor, surfactant, diluent, excipient, pH adjuster, soothing agent, buffer, sulfur-containing reducing agent, oxidation inhibitor, and so forth.

The suspending agent includes methyl cellulose, polysorbate 80, hydroxyethyl cellulose, gum Arabic, tragacanth powder, sodium carboxymethyl cellulose, and polyoxyethylene sorbitan monolaurate.

The solubilizer includes polyoxyethylene hydrogenated castor oil, polysorbate 80, nicotinamide, polyoxyethylene sorbitan monolaurate, macrogol, and the ethyl esters of castor oil fatty acids.

The stabilizer includes dextran 40, methyl cellulose, gelatin, sodium sulfite, and sodium metasulfite.

Certain amino acids may also be added as a stabilizer (see, for example, Japanese Patent Application Laid-open Hei 10-182481). The amino acid to be added as stabilizer includes, for example, the free amino acid and salts thereof, such as odium salt, potassium salt, and hydrochloride. A single amino acid or a combination of two or more amino acids may be added. There are no particular limitations on the amino acid added as a stabilizer, but preferred amino acids in this regard are, for example, leucine, tryptophan, serine, glutamic acid, arginine, histidine, and lysine.

The isotonizer includes D-mannitol and sorbitol.

The preservative includes methyl para-hydroxybenzoate, ethyl para-hydroxybenzoate, sorbic acid, phenol, cresol, and chlorocresol.

The adsorption inhibitor includes human serum albumin, lecithin, dextran, ethylene oxide propylene oxide copolymer, hydroxypropyl cellulose, methyl cellulose, polyoxyethylene hydrogenated castor oil, and polyethylene glycol.

Representative examples of the surfactant are nonionic surfactants, for example, nonionic surfactants having an HLB of 6 to 18, e.g., the fatty acid esters of sorbitan, such as sorbitan monocaprylate, sorbitan monolaurate, and sorbitan monopalmitate; the fatty acid esters of glycerol, such as glycerol monocaprylate, glycerol monomyristate, and glycerol monostearate; the fatty acid esters of polyglycerol, such as decaglyceryl monostearate, decaglyceryl distearate, and decaglyceryl monolinolate; polyoxyethylene sorbitan fatty acid esters, such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbitol fatty acid esters, such as polyoxyethylene sorbitol tetrastearate and polyoxyethylene sorbitol tetraoleate; polyoxyethylene glycerol fatty acid esters, such as polyoxyethylene glyceryl monostearate; polyethylene glycol fatty acid esters, such as polyethylene glycol distearate; polyoxyethylene alkyl ethers, such as polyoxyethylene lauryl ether; polyoxyethylene-polyoxypropylene alkyl ethers, such as polyoxyethylene-polyoxypropylene glycol, polyoxyethylene-polyoxypropylene propyl ether, and polyoxyethylene-polyoxypropylene cetyl ether; polyoxyethylene alkylphenyl ethers such as polyoxyethylene nonylphenyl ether; polyoxyethylene castor oil and polyoxyethylene hardened castor oil (polyoxyethylene hydrogenated castor oil); polyoxyethylene/beeswax derivatives such as polyoxyethylene sorbitol beeswax; polyoxyethylene/lanolin derivatives such as polyoxyethylene lanolin; and polyoxyethylene fatty acid amides such as polyoxyethylene stearamide. Additional representative examples of the surfactant are anionic surfactants, for example, alkyl sulfates that have C₁₀₋₁₈ alkyl groups, such as sodium cetyl sulfate, sodium lauryl sulfate, and sodium oleyl sulfate; polyoxyethylene alkyl ether sulfates that have an average ethylene oxide addition of 2 to 4 moles and a C₁₀₋₁₈ alkyl group, such as sodium polyoxyethylene lauryl sulfate; and alkyl sulfosuccinate ester salts having C₈₋₁₈ alkyl groups, such as sodium lauryl sulfosuccinate. Additional representative examples of the surfactant are natural surfactants, for example, lecithin, glycerophospholipids, sphingophospholipids such as sphingomyelin, and sucrose fatty acid esters with C₁₂₋₁₈ fatty acids. A single one of these surfactants or a combination of two or more of these surfactants can be added to the formulation of the present invention. Polyoxyethylene sorbitan fatty acid esters, such as polysorbate 20, 40, 60, and 80, are preferred surfactants, and polysorbate 20 and 80 are particularly preferred. Polyoxyethylene-polyoxypropylene glycols such as poloxamer (e.g., Pluronic F-68 (registered trademark)) are also preferred.

The sulfur-containing reducing agent includes sulfhydryl group-containing reducing agents such as N-acetylcysteine, N-acetylhomocysteine, thiolactic acid, thiodiglycol, thioethanolamine, thioglycerol, thiosorbitol, thioglycol acid and its salts, sodium thiosulfate, glutathione, and C₁₋₇ thioalkanoic acids.

The oxidation inhibitor includes erythorbic acid, dibutylhydroxytoluene, butylhydroxyanisole, α-tocopherol, tocopherol acetate, L-ascorbic acid and its salts, L-ascorbic acid palmitate, L-ascorbic acid stearate, sodium bisulfite, sodium sulfite, triamyl gallate, and propyl gallate and chelating agents such as disodium ethylenediaminetetraacetate (EDTA), sodium pyrophosphate, and sodium metaphosphate.

Components that may be added as appropriate also include inorganic salts such as sodium chloride, potassium chloride, calcium chloride, sodium phosphate, potassium phosphate, and sodium bicarbonate, and organic salts such as sodium citrate, potassium citrate, and sodium acetate.

The content of all patents and reference documents expressly cited in the specification of this application are hereby incorporated by reference in its entirety. In addition, the content of the specification and drawings of Japanese Patent Application 2006-158998, which is the basis for the priority claim of this application, are hereby incorporated by reference in its entirety.

The present invention is described in detail by the Examples below, but is not limited by those examples.

### Example 1

### Mobilization of hematopoietic cells by EPO

Recombinant human EPO (Epogin (registered trademark), active ingredient: epoetin beta) was administered subcutaneously to mice (C57BL/6, age: 9 weeks) once a day for 4 days at a dose of 100 µg/kg/day. Vehicle alone was similarly administered subcutaneously to the control mice. The mice were sacrificed on day 5 and peripheral blood cells (0.6 to 1 mL) were collected. Red blood cell lysing buffer (SIGMA) containing 8.3 g/L ammonium chloride in 0.01 M Tris-HCl buffer pH 7.5 ± 0.2 was added as hemolyzing agent and let stand for 5 to 10 minutes at room temperature, followed by washing with 2% BSF+PBS(-). CD16/CD32 (Fcγ III/II receptor) nonspecific reaction blocking antibody (BD-Pharmingen, San Diego, CA) was added at 1 µg/10⁶ cells and kept on ice for 30 minutes. FITC-conjugated anti-mSca-1 antibody (BD Pharmingen, San Diego, CA), PE-conjugated anti-mCD34 antibody, APC-conjugated anti-mc-Kit antibody (BD Pharmingen, San Diego, CA), and a cocktail of APC-Cy7-conjugated anti-mCD3, CD4, CD8a, CD45R/B220, Ly6G(Gr-1), CD11b(Mac-1 α chain), and Ter119 antibodies were used as lineage markers. After antibody staining, the cells were analyzed by FACSvantage SE (Becton Dickinson, Mountain View, CA).

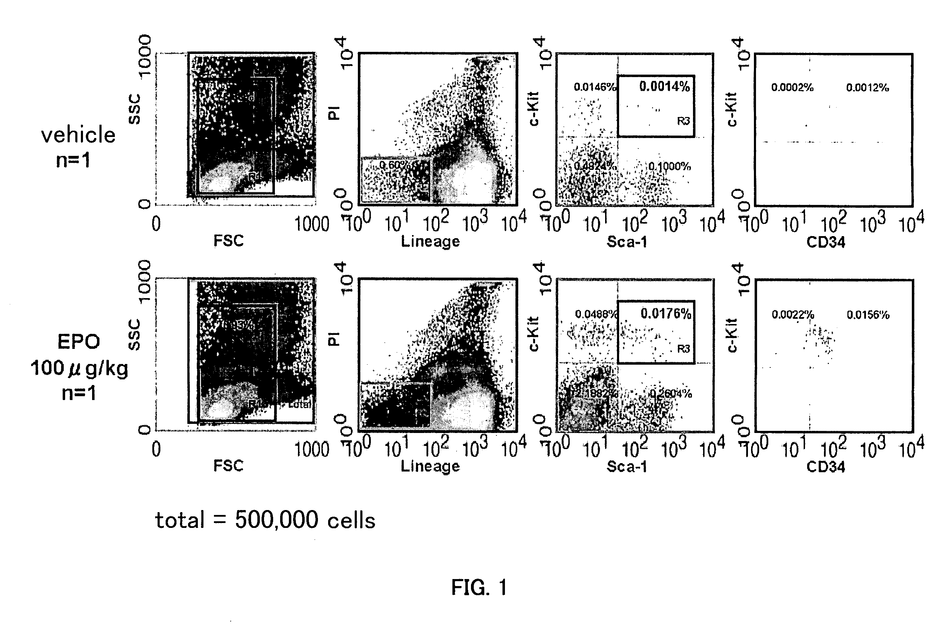

The results are shown in Fig. 1. As shown in R3 in Fig. 1, the number of c-Kit⁺Sca-1⁺Lin- cells was increased by about 13-fold by the administration of EPO. As c-Kit⁺Sca-1⁺Lin- cells are a marker of CFU-S-like cells in mice (see, for example, Blood, 1992 Dec. 15;80(12):3044-50). The results reported above demonstrate that CFU-S-like cells are mobilized into the peripheral blood by administration of EPO.

### CFU-S colony assay

Peripheral blood cells from C57/b6 mice that had received EPO subcutaneously for 5 days were used as the donor cells. The donor cells of 10⁴ to 10⁵ cells/100-200 µL were transplanted via the tail vein of the recipient C57/b6 mice that had received a lethal dose of γ-radiation (950 cGy from ¹³⁷Cs) using a radiological exposure instrument (Hitachi Medico). The spleen was resected 8 or 12 days after transplantation and was analyzed for spleen colonies by Bouin's staining (number of colonies formed in the spleen).

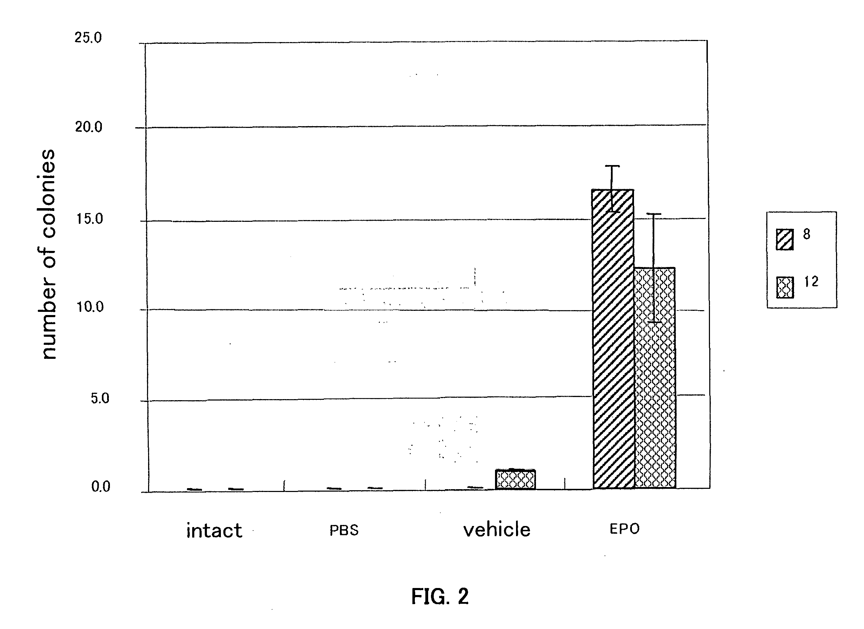

The results are shown in Fig. 2. consistent to the results from the FACS analysis, the mobilization of CFU-S-like cells into the peripheral blood was observed.

### Example 2

### Angiogenesis therapy by transplantation of peripheral blood mononuclear cells

This study was designed to investigate whether hematopoietic stem cells could be mobilized into the peripheral blood in 5 patients with severe ischemic extremity disease by the administration of an erythropoietin formulation. A clinical study of angiogenesis therapy by the autologous transplantation of peripheral mononuclear cells was also carried out. An open-labeled study protocol was employed for the general design of the clinical study.

The patients received 6000 U EPO by subcutaneous injection two weeks prior to the planned cell transplantation (first administration). One week prior to cell transplantation, blood was drawn (about 400 mL) for the purpose of autologous blood donation and 6000 U EPO was injected subcutaneously (second administration). On the morning of the day of the operation, 6000 U EPO was injected (third administration), followed by the separation of about 10⁹ peripheral mononuclear cells from the peripheral blood by blood apheresis. Peripheral blood samples were collected prior to EPO administration, after the second administration, and immediately before apheresis and were subjected to blood testing (WBC, CRP, CK) and the CD34-positive cell count.

The separated peripheral mononuclear cells were injected at 50 to 100 sites into the muscle of the patient's ischemic limb or limbs. The effect of the angiogenesis therapy was evaluated based on observation and measurement of the following items prior to cell transplantation, on the day following an administration, after one week, two weeks, 1 month, 2 months, and 6 months.

QOL: pain was evaluated on a visual analogue scale (VAS), with 0 being no pain and 10 being the most intense pain
blood sampling
blood testing
angiography
treadmill testing: the absolute walking distance or pain appearance distance were measured at 2.4 km/hr, horizontal
objective evaluation of skin and ulcer lesions: ankle-brachial pressure index (ABPI), digital plethysmography, treadmill test (ambulatory capacity), thermography, laser doppler (LDPI), and transdermal oxygen partial pressure (TdPO₂) measurement

### Changes in CD34-positive cells

A summary of the case, transplanted cell count, and CD34-positive cell count are given in Table 1 below. As normalized with the value prior to erythropoietin administration as 100%, the proportion of CD34-positive cells in the peripheral blood showed an increase of 82 to 245% (average 158%) at the blood draw one week after the first administration of erythropoietin. The increase was 88 to 175% (average 139%) immediately prior to apheresis after an additional week. These results indicates that the CD34-positive cell count (during phlebotomy) at one week after the first erythropoietin administration is the same as or greater than the cell count after two weeks (immediately before apheresis).

CD34-positive cells accounted for 0.02 to 0.1% (average = 0.06%) of the mononuclear cells recovered by blood apheresis.

**Table 1.**

| case | sex | ag e | causal disease | site of transplantation | total transplanted cells | CD34-positive cells |
|---|---|---|---|---|---|---|
| 1 | male | 77 | arteriosclerosis | right lower limb | 0.5 × 10⁹ | 0.1 × 10⁶ |
| | | | obliterans | | | (0.2 × 10⁴ cells/kg) |
| 2 | male | 66 | arteriosclerosis | left lower limb | 16.7 × 10⁹ | 5.0 × 10⁶ |
| | | | obliterans | | | (8 × 10⁴ cells/kg) |
| 3 | male | 48 | Buerger's | both upper limbs | 9.2 × 10⁹ | 9.2 × 10⁶ |
| | | | disease | | | (14 × 10⁴ cells/kg) |
| 4 | male | 48 | Buerger's | right upper limb | 6.9 × 10⁹ | 5.5 × 10⁶ |
| | | | disease | | | (9 × 10⁴ cells/kg) |

### Clinical evaluation of cell transplantation

### Evaluation by subjective symptoms and angiography

**Table 2.**

| case | observation period | condition | VAS | Fontatine classification | angiography |
|---|---|---|---|---|---|
| 1 | 11 months | numbness rest pain | 7 -> 7 | III -> II | no change |
| 2 | 3 months | numbness rest pain | 2 -> 0 | III -> II | no change |
| 3 | 3 months | ulcers | 6→3 | not classifiable | - |
| 4 | 1 week | ulcers | 3→1 | not classifiable | not done |

From a subjective standpoint, an improvement in pain was observed in 3 out of 4 cases.

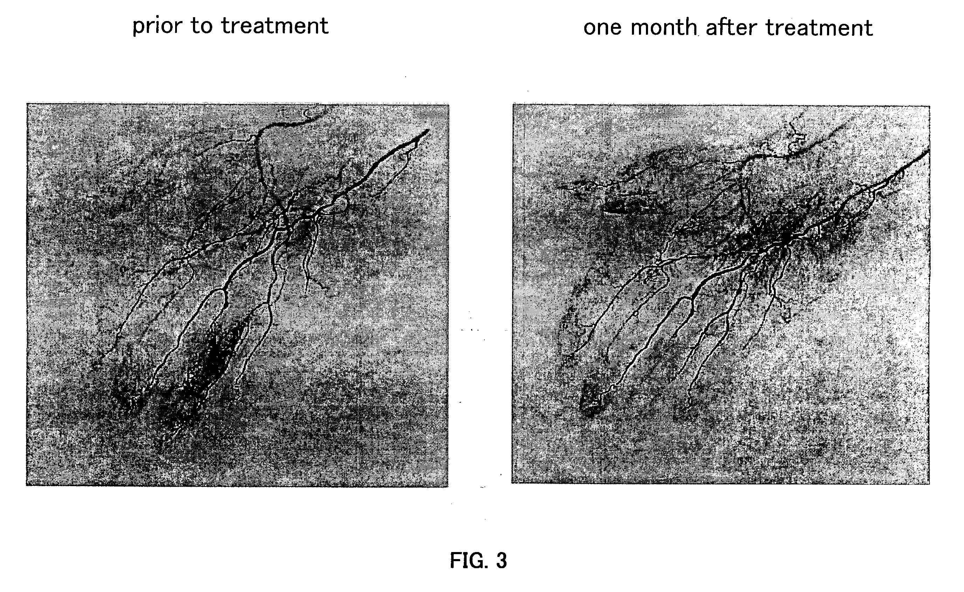

In addition, revascularization was observed in angiography in case 3 at one month after transplantation (Fig. 3).

### Objective evaluation

**Table 3.**

| case | observation period | ABPI | digital plethysmography | thermogram | LDPI | TdPO₂ |
|---|---|---|---|---|---|---|
| 1 | 11 months | 0.24 -> 0.4 | improved | moderately improved | no change | no change |
| 2 | 3 months | 0.49 -> 0.66 | improved | no change | no change | no change |
| 3 | 3 months | not done | improved | moderately improved | moderately improved | improved |
| 4 | 1 week | not done | improved | not done | not done | not done |

Improvements in the ABPI and TdPO₂ were noted in some cases. A significant improvement in the walking distance in the treadmill test was seen in case 2, from 160 m to 915 m.

The preceding results showed that hematopoietic stem cells could be mobilized into the peripheral blood by the administration of erythropoietin and that peripheral occlusive artery diseases could be treated by angiogenesis therapy via the transplantation of peripheral mononuclear cells into the ischemic skeletal muscle.

Moreover, the CD34-positive cell content in the peripheral blood mononuclear cells recovered at one week after the first EPO administration was either equal to or greater than that in the mononuclear cells recovered after two weeks, indicating that the mononuclear cells recovered at one week after EPO administration are able to provide an equal or greater angiogenetic therapeutic effect than the mononuclear cells recovered after two weeks.

### Industrial Applicability

The methods and composition of the present invention are useful for the treatment of ischemic diseases, such as peripheral vascular disorder.

## Claims

1. A composition for mobilizing mononuclear cells for use in the treatment of ischemic diseases into peripheral blood, comprising erythropoietin as an active ingredient.

2. A composition for mobilizing mononuclear cells for use in stimulating revascularization into peripheral blood, comprising erythropoietin as an active ingredient.

3. The composition according to claim 1 or 2, wherein the mobilized mononuclear cells are collected from a subject and then administered to the subject.

4. The composition according to any one of claims 1-3, which is administered to a subject from 3 to 12 days prior to collecting peripheral blood from the subject.

5. A composition for the treatment of ischemic diseases, comprising as an active ingredient mononuclear cells separated from peripheral blood from a subject to whom erythropoietin has previously been administered.

6. A composition for stimulating revascularization, comprising as an active ingredient mononuclear cells separated from peripheral blood from a subject to whom erythropoietin has previously been administered.

7. The composition according to claim 5 or 6, wherein the erythropoietin is administered from 3 to 12 days prior to collecting peripheral blood.

8. A composition for mobilizing CD34-positive cells for use in the treatment of ischemic diseases into peripheral blood, comprising erythropoietin as an active ingredient.

9. A composition for mobilizing CD34-positive cells for use in stimulating revascularization into peripheral blood, comprising erythropoietin as an active ingredient.

10. A method of preparing mononuclear cells for transplantation, comprising the steps of:
(a) administering erythropoietin to a subject; and
(b) collecting peripheral blood mononuclear cells from the subject about one week after the erythropoietin administration.

11. A method for treating an ischemic disease in a subject comprising the steps of:
(a) administering erythropoietin to the subject;
(b) collecting peripheral blood mononuclear cells from the subject; and
(c) administering the collected peripheral blood mononuclear cells to a target site of the subject.

12. A method for stimulating revascularization in a subject comprising the steps of:
(a) administering erythropoietin to the subject;
(b) collecting peripheral blood mononuclear cells from the subject; and
(c) administering the collected peripheral blood mononuclear cells to a target site of the subject.
